# EUROPEAN PATENT APPLICATION

(11) **EP 1 329 235 A2**
(43) Date of publication of application: **23.07.2003**
(21) Application number: 03450003.3
(22) Date of filing: 08.01.2003
(51) Int. Cl.: A61M 5/42, A61M 5/30

(54) **Device for delivering liquid medications or nutrients and gases to local tissue**

(30) Priority: 09.01.2002 US 43505
(71) Applicant: Johnson, Lanny L., Okemos, Michigan 48864 (US)
(72) Inventor: Johnson, Lanny L., Okemos, Michigan 48864 (US)
(74) Representative: Haffner, Thomas M.

(57) **Abstract**

A delivery member (16) is detachably secured to a discharge port (14) of a syringe (10). The delivery member (16) is provided with a fluted, open-ended distal portion having a cross-sectional area which is substantially greater than that of the discharge port (14). When the open end of the fluted portion is pressed against tissue (26) and liquid medication or nutrient, or gas, is supplied under pressure to the delivery member (16) from the syringe (10), the liquid is perfused into the tissue.

## Description

### BACKGROUND OF THE INVENTION

### 1. Field of the Invention

The present invention relates to apparatus for delivering liquid medications or nutrients, or gases, to local tissue by pressure perfusion.

### 2. Prior Art

Medication or nutrient agents typically are delivered by oral ingestion, subcutaneous or intramuscular injection, and intravenously. As alternatives to such delivery devices, various "needleless" hypodermic injection devices have been developed. Such devices are characterized by the delivery of medication or nutrients under high pressure in a very localized area, and they customarily are complex in construction.

### SUMMARY OF THE INVENTION

The present invention is a non-invasive delivery device particularly suitable for directing gases or liquid medications or nutrients to tissue not provided with an active blood supply. Such avascular tissue (examples of which are cartilage and the cornea) is largely composed of water, and it readily takes up fluid by diffusion. The invention is not limited to use with avascular tissue, however. It can be used to deliver medication or nutrient agents, or gases, to circular or tubular tissue such as nerve, artery, vein, bowel, etc. Also, the present delivery device can be used for healing purposes and in the destruction of unwanted tissue or tumor.

Briefly, the invention comprises a fluted delivery member which can be secured to a conventional syringe to receive liquid or gas discharged from the syringe when its plunger is actuated. The interior of the fluted member includes a bell-shaped chamber portion. With the open end of the fluted member pressed against or secured to the area to which the liquid or gas is to be delivered, and with the syringe's plunger depressed, the chamber fills with liquid or gas discharged from the syringe. When the chamber becomes full and the plunger continues to be depressed, the liquid or gas becomes sufficiently pressurized to enter the tissue by perfusion and to diffuse throughout the neighboring tissue. This may be calibrated and also simultaneously read by ultrasound imaging as to the depth and width of penetration ofthe liquids or gas.

### BRIEF DESCRIPTION OF THE DRAWINGS

The invention now will be described in further detail with respect to the accompanying drawings, wherein:
FIG. 1 is a perspective view of a first embodiment of the invention; and
FIG. 2 is a fragmented perspective view of a second embodiment of the invention.

### DETAILED DESCRIPTION OF PREFERRED EMBODIMENTS OF THE INVENTION

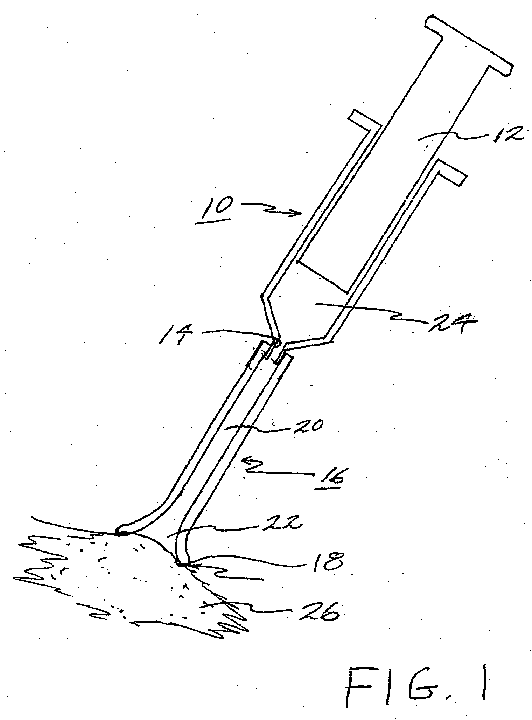

Referring now to the drawings, FIG. 1 illustrates a first embodiment of the invention. A source of liquid or gas, such as a conventional syringe 10 provided with a plunger 13 and a discharge port 14, is detachably secured to a delivery member 16. The delivery member comprises a generally cylindrical proximal end and a fluted open distal end. Outer edge 18 of the fluted end is rounded.

The interior of delivery member 16 serves as a chamber 20 for receiving liquid or gas, such as oxygen or nitric oxygen, discharged from the syringe 10. The fluted distal end defines a bell-shaped chamber portion 22 having a discharge area, defined by edge 18, which is substantially greater than that of the syringe's discharge port 14.

In operation, portion 24 of the syringe is filled in conventional fashion with a liquid containing a medication or nutrient agent or with a gas. The volume ofthe liquid or gaseous fluid introduced within portion 24 is greater than the interior volume of delivery member 16. Member 16 is then secured to the syringe's discharge port 14, and its rounded edge 18 is pressed into sealing relationship against the tissue 26 to which the liquid or gas is to be directed. On depression ofthe syringe's plunger 12, the liquid or gas is transferred through port 14 into chamber 20. Once the chamber is filled, and with pressure still being applied to plunger 12, the liquid or gas is pressurized within chamber 20, and portion 22 in particular, causing perfusion of the liquid or gas into the tissue over the entire area defined by edge 18 of the delivery member 16. If desired, the pressure within chamber 20 can be calibrated.

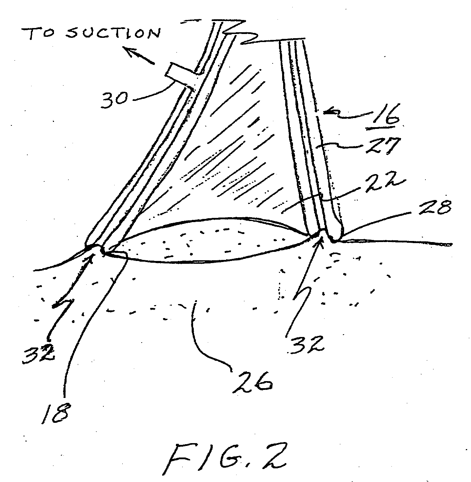

In order to improve the seal between chamber portion 22 of delivery member 16 and tissue 26, member 16 is provided with an additional wall 27 (Fig. 2) which is spaced from the inner wall and which also has a rounded outer edge 28. Wall 27 is provided with a port 30 for attachment to a suction device (not shown). The application of suction while member 16 is pressed against tissue causes a bead of tissue -- shown as 32 -- to be drawn up between edges 18 and 28 to form a seal to prevent leakage from chamber portion 22.

## Claims

1. A delivery device for liquid medication or nutrients, or gas, comprising:
a source of said liquid or gas; and
a delivery member detachably joined to a discharge port of said source, said member including a fluted, open-ended distal portion having a cross-sectional area substantially greater than a cross-sectional area of the discharge port for delivering pressurized liquid or gas supplied by the source into tissue by means of perfusion.

2. A device according to Claim 1, wherein said fluted portion has a rounded outer edge.

3. A device according to Claim 1, wherein said delivery member comprises spaced inner and outer walls at said distal portion of the delivery member.

4. A device according to Claim 3, wherein said inner and outer walls are rounded at outer edges thereof.

5. A device according to Claim 3, wherein said outer wall includes a suction port.

6. A device according to Claim 5, wherein said inner and outer walls are rounded at outer edges thereof.

7. A device according to Claim 1, wherein said source is a syringe.
